# FASCICULE DE BREVET EUROPEEN

(11) **EP 1 351 627 B1**
(45) Date de publication et mention de la délivrance du brevet: **18.08.2004**
(21) Numéro de dépôt: 02711984.1
(22) Date de dépôt: 17.01.2002
(51) Int. Cl.: A61F 2/14

(54) **ELEMENT CORRECTEUR DE LA PRESBYTIE**
ELEMENT ZUR PRESBYOPIEKORREKTUR
PRESBYOPIA CORRECTING ELEMENT

(30) Priorité: 19.01.2001 FR 0100703
(43) Date de publication de la demande: 15.10.2003
(73) Titulaire: Baikoff, Georges, 13007 Marseille (FR)
(72) Inventeur: Baikoff, Georges, 13007 Marseille (FR)
(74) Mandataire: Boutin, Antoine
(86) Numéro de dépôt international: PCT/FR2002/000179
(87) Numéro de publication internationale: WO 2002/056801

(56) Documents cités:
- EP-A- 1 125 560
- FR-A- 2 784 287
- FR-A- 2 787 991

## Description

La présente invention concerne d'une manière générale la correction de la vision par l'insertion d'un élément correcteur dans l'oeil et plus précisément la correction de la presbytie.

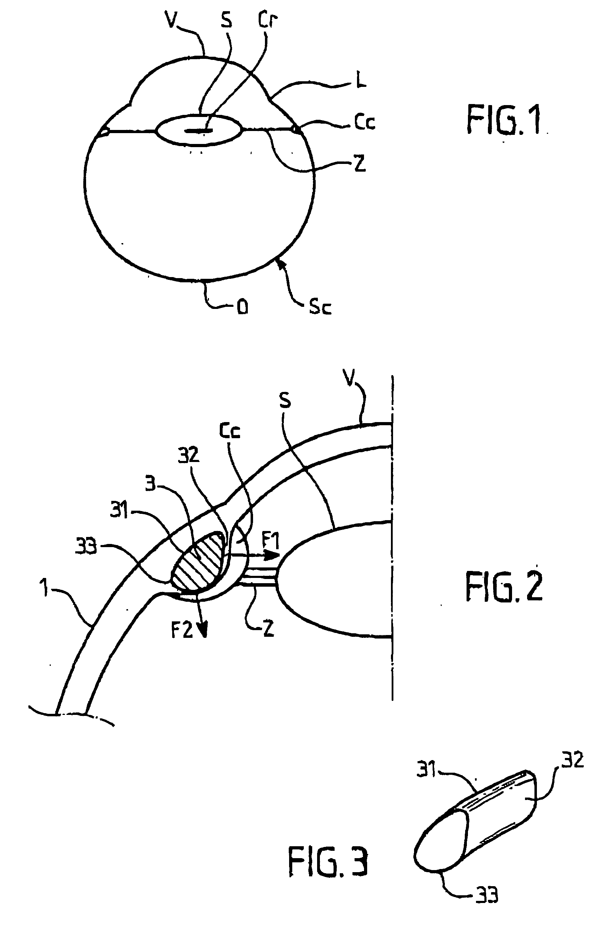

Rappelons que, comme représenté à la figure 1, le cristallin Cr enfermé dans le sac cristallinien S est suspendu au corps ciliaire Ce par l'intermédiaire du ligament zonulaire Z (ou zonule). Ce corps ciliaire Cc tapisse la face interne de la sclère suivant un anneau situé en moyenne à une latitude distante de 2 à 3 mm du limbe, si l'on prend l'axe optique pour référence.

La presbytie est une perte ou une réduction du pouvoir accommodatif de l'oeil qui se produit lorsque le sujet vieillit.

Dans une théorie datant d'une centaine d'années environ, Von Helmholtz expliquait la physiologie de l'accommodation en vision de près par un relâchement des tensions zonulaires exercées sur le cristallin lors de la contraction du corps ciliaire, Ce relâchement des tensions entraîne une reprise par le cristallin d'une forme plus globuleuse présentant des rayons de courbure plus faibles donc plus convergents permettant la mise au point. Dans le même temps, le cristallin se déplace vers l'avant dans un plan antéro-postérieur.

Pour sa part, Schachar a proposé depuis 1992, notamment dans ses brevets US 5 354 331, US 5 465 737, US 5 489 299 et US 6 007 578 une théorie contraire à celle de Von Helmholtz selon laquelle l'accommodation serait due à un effort de traction exercé sur le cristallin lorsque le corps ciliaire se relâche, un tel effort mettant en tension le ligament zonulaire qui créerait un aplatissement de la périphérie du cristallin et un bombement du centre de celui-ci.

De plus, selon Schachar, le diamètre du cristallin augmente au cours du vieillissement et la distance séparant la périphérie du cristallin du corps ciliaire diminue peu à peu ce qui conduit à un relâchement de la zonule. Il en résulterait que l'effort centrifuge exercé par le corps ciliaire sur la périphérie du cristallin n'est plus assez important pour assurer l'accommodation.

Il a par exemple été proposé dans le FR 2 787 991 d'accroître l'effort exercé par les zonules sur la périphérie du cristallin en introduisant dans l'oeil un dispositif de correction globalement annulaire positionné dans la zone du sulcus ciliaire. Un tel dispositif est en appui contre la paroi de l'oeil par son bord externe, dans la zone du sulcus ciliaire, et en appui contre la zone médiane des zonules par son bord interne. L'action du bord interne sur les zonules déplace la partie médiane de celles-ci vers l'arrière de l'oeil, Il en résulte un déplacement de la zone médiane des zonules vers l'arrière de l'oeil dans la direction de l'axe optique et vers l'extérieur dans le sens perpendiculaire à l'axe optique ce qui a pour effet une augmentation du diamètre du cristallin.

Schachar a proposé dans les brevets cités ci-dessus différentes méthodes permettant d'améliorer le pouvoir d'accommodation d'un oeil, consistant par exemple à réduire chirurgicalement la longueur des zonules ou le diamètre du cristallin, ou d'empêcher le grossissement du cristallin.

Une autre méthode de traitement proposée par Schachar a été très largement mise en oeuvre. Elle consiste à traiter la presbytie en positionnant un anneau tronconique autour de l'anneau scléral pour créer une sorte de suspension externe, afin d'agrandir le diamètre du corps ciliaire et donc de retendre la zonule, voir par exemple le document EP1125560A compris dans l'état de la technique tel que défini à l'article 54(3) CBE.

Une telle intervention étant assez lourde, il a été proposé par la suite de positionner sur la sclère des segments d'expansion sclérale arqués, de rayon de courbure inférieur au rayon de courbure de la sclère. Ces segments traversent des passants incisés dans la surface de la sclère concentriquement au limbe, à l'aplomb du corps ciliaire, et sont en appui par leurs extrémités sur la surface externe de la sclère.

Dans le FR 98 12834 du demandeur, de tels segments présentent des extrémités de forme spatulée afin de ne pas risquer d'induire une perforation de la sclère aux points d'appui du segment sur la sclère et d'empêcher le retournement desdits segments.

Il a été constaté que les opérations effectuées depuis 1992 en suivant la théorie de Schachar ont tantôt bien réussi et permis de rendre au patient une bonne vue, tantôt totalement échoué, l'accommodation n'étant pas réellement meilleure après l'intervention.

De plus, chez certains patients, les segments ont été expulsés de la sclère, après déchirement des passants sous lesquels ils étaient positionnés.

Il en résulte que la théorie de Schachar n'est pas en mesure de résoudre le problème du traitement de la presbytie.

En analysant les résultats des interventions pratiquées par les spécialistes, le demandeur est arrivé à la conclusion que la théorie de Schachar serait inexacte bien que sa mise en oeuvre permette dans certains cas d'obtenir le résultat recherché.

Il en a conclu que la correction de la presbytie observée pour certains patients après l'implantation d'un anneau tronconique ou de segments arqués sur la sclère ne serait pas due comme l'avait pensé Schachar à un effet de traction exercé sur la zonule et donc sur le cristallin, mais à un résultat induit obtenu lors de l'intervention sans que l'opérateur le recherche.

Le demandeur a repris en compte le fait que le globe oculaire a une surface souple mais inextensible et en a conclu qu'il n'est pas possible d'augmenter la circonférence de l'anneau scléral en exerçant une tension centrifuge en certains points de la sclère.

En examinant plus en détail les interventions réalisées suivant la méthode Schachar, le demandeur a constaté qu'en exerçant une tension centrifuge en certains points, la mise en place des segments d'expansion sclérale exerçait en réaction une pression centripète au droit de leurs points d'appui sur la sclère.

Le demandeur explique alors l'effet constaté avec certains anneaux ou segments d'expansion scléraux non pas par la traction qu'ils exercent sur la zonule en certains points mais par la pression qu'ils exercent sur celle-ci en d'autres points. Lorsque la pression est exercée au droit du corps ciliaire, elle compense artificiellement le défaut de contraction dudit corps ciliaire et aide l'oeil dans son travail d'accommodation tel qu'il a été décrit par Von Helmholtz.

Ces observations conduisent le demandeur a proposer dans la présente invention un élément correcteur permettant de corriger la presbytie de manière reproductible et non plus aléatoire comme dans les interventions proposées depuis près de dix ans.

A cet effet, l'invention concerne un élément correcteur de la presbytie destiné à être implanté dans l'oeil au droit du corps ciliaire, caractérisé en ce qu'après sa mise en place dans la partie profonde de la sclère, il présente en section une paroi externe parallèle à la surface de la sclère et une paroi interne parallèle à l'axe optique de l'oeil, de sorte qu'il exerce sur ledit corps ciliaire un effort centripète dirigé perpendiculairement à l'axe optique.

La sclère étant inextensible, un tel élément agit en prenant appui sur la portion de sclère qui le recouvre pour repousser le corps ciliaire en direction de l'axe optique de l'oeil et former des indentations dans ledit corps ciliaire. Il en résulte des efforts centripètes sur ledit corps ciliaire compensant artificiellement la perte de contraction de celui-ci et rétablissant ainsi son action nécessaire à l'accommodation, c'est-à-dire un relâchement zonulaire.

L'augmentation de l'effet de contraction du corps ciliaire avec diminution du diamètre de l'anneau ciliaire par des éléments formant des indentations dans le corps ciliaire permet de relâcher la zonule et de laisser le cristallin profiter de sa souplesse résiduelle pour devenir plus globuleux.

L'élément correcteur selon l'invention est encore remarquable en ce que :
- il est constitué par un segment arqué dont le rayon de courbure est tel qu'après sa mise en place dans l'oeil, ledit segment soit centré sur l'axe optique dudit oeil,
- une paroi arrière raccorde les extrémités arrière éloignées l'une de l'autre desdites parois externe et interne,
- un arrondi raccorde les extrémités avant concourantes desdites parois externe et interne,
- l'angle compris entre la paroi externe et la paroi interne est de l'ordre de 45°,
- la paroi arrière est arrondie,
- la paroi arrière est constituée par une portion de tore.

L'invention sera mieux comprise grâce à la description qui va suivre donnée à titre d'exemple non limitatif en référence aux dessins annexés dans lesquels :
- la figure 1 et une vue schématique en coupe d'un oeil,
- la figure 2 est une vue partielle en coupe d'un oeil dans lequel est implanté un élément correcteur selon l'invention,
- la figure 3 est une vue en perspective d'un élément correcteur suivant l'invention.

Sur la figure 2, on a schématisé en 1 la surface de la sclère au droit du corps ciliaire et en 2 l'axe optique de l'oeil.

Les expressions avant et arrière sont définies par rapport à l'oeil.

Les figures 2 et 3 montrent un élément 3 suivant une forme de réalisation préférée de l'invention. Cet élément est constitué par un segment arqué dont le rayon de courbure est tel qu'il soit centré sur l'axe optique 2 de l'oeil lorsqu'il est mis en place, et donc tel que ledit segment soit parallèle à l'anneau scléral au niveau de l'insertion du muscle ciliaire, Ledit segment arqué a par exemple une longueur de l'ordre de 4 à 5 millimètres et un rayon de courbure de l'ordre de 7 millimètres.

Vu en coupe, l'élément 3 a globalement la forme d'un triangle. Il comporte une paroi externe 31 destinée à être disposée parallèlement à la surface 1 de la sclère, une paroi interne 32 destinée à être disposée parallèlement à l'axe optique 2 de l'oeil.

Compte tenu de la géométrie de l'oeil, ces parois externe 31 et interne 32 de l'élément 3 définissent entre elles un angle de l'ordre de 45°.

Lesdites parois externe 31 et interne 32 ont des extrémités concourantes vers l'avant de l'élément 3 et éloignées l'une de l'autre vers l'arrière.

Une paroi arrière 33 raccorde les extrémités arrière éloignées des parois externe 31 et interne 32 et un arrondi raccorde les extrémités avant concourantes des deux parois.

De manière connue en soi, la sclère est un corps semi-rigide et inextensible alors que le corps ciliaire est un tissu mou. Un corps étranger introduit entre la sclère et le corps ciliaire ne peut donc pas déformer la sclère mais engendre une déformation du corps ciliaire. Il en résulte que lorsqu'il est mis en place dans l'oeil, au droit du corps ciliaire, l'élément 3 prend appui par sa paroi externe 31 sur la portion inextensible de sclère qui le recouvre pour exercer sur le corps ciliaire Cc un effort centripète F1 orienté perpendiculairement à la paroi interne 32, donc perpendiculairement à l'axe optique 2, et dirigé vers ledit axe optique 2. Un tel effort agit directement sur le corps ciliaire Cc et exerce sur celui-ci une pression qui le repousse en direction de l'axe optique 2 de l'oeil ce qui crée artificiellement un déplacement de celui-ci semblable à celui qui s'opère lors d'une contraction dudit corps ciliaire. Ce déplacement a pour effet de relâcher la zonule Z, dans l'axe de celle-ci. Cet effet compense la perte de pouvoir de contraction du corps ciliaire et permet au cristallin de prendre une forme plus globuleuse présentant des rayons de courbure plus faibles.

L'extrémité avant de l'élément 3 est de préférence arrondie de manière à éviter de blesser les tissus de l'oeil.

La paroi arrière 33 peut être prévue plus ou moins arrondie.

De manière préférée, ladite paroi arrière 33 est prévue de manière à donner à la section de l'élément correcteur 3 une forme semblable à une goutte, à cet effet, elle peut être constituée par une portion de tore. Une telle réalisation permet d'améliorer encore la correction engendrée par l'élément correcteur 3 en fournissant un effort de pression F2 exercé sur le corps ciliaire Cc par la paroi arrière 33.

L'effort F2 est orienté vers le centre et vers l'arrière de l'oeil, il assure une compression du vitré et induit une poussée, et donc un déplacement du cristallin vers l'avant semblable à celui décrit par Von Helmholtz.

L'élément correcteur 3 selon l'invention est placé à la face profonde du plan scléral pour profiter au mieux des effets dus à l'inextensibilité de la sclère et près de la surface du corps mou constitué par le corps ciliaire, à travers des incisions de faibles dimensions pratiquées dans la sclère, ou entre la sclère et le corps ciliaire comme représenté au dessin.

Afin d'obtenir un effet maximal, la mise en place de l'élément suivant l'invention doit être réalisée en effectuant une incision dans la sclère sur une profondeur d'au moins 600 microns et en réalisant à cette profondeur un tunnel borgne s'étendant parallèlement à la surface de la sclère. Une échographie pourra être effectuée avant l'opération afin de vérifier l'épaisseur de la sclère pour ne pas risquer de blesser le corps ciliaire. Après insertion de l'élément correcteur, l'incision est suturée pour que l'élément reste positionné profondément dans la sclère.

Pour traiter la presbytie, plusieurs éléments sont répartis dans l'oeil, autour du corps ciliaire afin de constituer plusieurs zones d'appui réparties autour du cristallin et induire sur celui-ci des effets proches de ceux qu'il supporte lors de la contraction d'un corps ciliaire jeune. On positionnera par exemple de 3 à 8 segments selon l'invention dans l'oeil du patient, de préférence 4 segments disposés à 90° les uns par rapport aux autres.

L'élément correcteur 3 est de préférence prévu pour avoir en section une longueur parallèle à l'axe optique telle qu'il soit en appui sur le corps ciliaire même s'il n'est pas parfaitement bien positionné au droit de celui-ci. A cet effet, la longueur de la paroi interne 32 est par exemple de l'ordre de 0,5 à 0,7 millimètre.

La longueur de la paroi externe 31 est par exemple de l'ordre de 0,7 à 1 millimètre, et l'épaisseur du segment 3, définie perpendiculairement à ladite paroi interne 32 est par exemple de l'ordre de 0,5 à 0,7 millimètre.

Suivant une forme de réalisation alternative non représentée au dessin, l'élément correcteur 3 selon l'invention est constitué par un anneau cylindrique disposé à l'intérieur de la sclère, autour du corps ciliaire.

Suivant encore une autre forme de réalisation non représentée au dessin, l'élément correcteur n'est pas de section uniforme sur toute sa longueur. Il peut présenter des zones à section triangulaire agissant sur le corps ciliaire et des zones de section plus faible n'ayant que très peu ou pas d'action sur le corps ciliaire. Une telle réalisation permet de créer un grand nombre de points d'appui sur le corps ciliaire afin de mieux répartir les efforts exercés sur celui-ci.

## Revendications

1. Elément correcteur de la presbytie destiné à être implanté dans un oeil au droit du corps ciliaire, **caractérisé en ce qu'**après sa mise en place dans la partie profonde de la sclère, il présente en section une paroi externe (31) parallèle à la surface de la sclère (1) et une paroi interne (32) parallèle à l'axe optique (2) de l'oeil, de sorte qu'il exerce sur ledit corps ciliaire un effort (F1) centripète dirigé perpendiculairement à l'axe optique (2) de l'oeil.

2. Elément correcteur selon la revendication 1, **caractérisé en ce qu'**il est constitué par un segment (3) arqué dont le rayon de courbure est tel qu'après sa mise en place dans l'oeil, ledit segment (3) soit centré sur l'axe optique dudit oeil.

3. Elément correcteur selon la revendication 1 ou selon la revendication 2, **caractérisé en ce qu'**une paroi arrière (33) raccorde les extrémités arrière éloignées l'une de l'autre desdites parois externe (31) et interne (32).

4. Elément selon l'une quelconque des revendications 1 à 3, **caractérisé en ce qu'**un arrondi raccorde les extrémités avant concourantes desdites parois externe (31) et interne (32).

5. Elément correcteur selon l'une quelconque des revendications 1 à 4, **caractérisé en ce que** l'angle compris entre la paroi externe (31) et la paroi interne (32) est de l'ordre de 45°.

6. Elément correcteur selon l'une quelconque des revendications 3 à 5, **caractérisé en ce que** la paroi arrière (33) est arrondie.

7. Elément correcteur selon l'une quelconque des revendications 3 à 6, **caractérisé en ce que** la paroi arrière (33) est constituée par une portion de tore.

## Patentansprüche

1. Element zur Korrektur von Weitsichtigkeit, das in ein Auge, im rechten Winkel zum Ziliarkörper, implantiert werden soll, **dadurch gekennzeichnet, daß** es nach seinem Einsetzen in den tiefen Teil der Sklera im Schnitt eine äußere Wand (31), die parallel zur Oberfläche der Sklera (1) verläuft, und eine innere Wand (32), die parallel zur optischen Achse (2) des Auges verläuft, aufweist, derart, daß es auf den Ziliarkörper eine zentripetalische Kraft (F1) ausübt, die senkrecht zur optischen Achse (2) des Auges gerichtet ist.

2. Korrigierendes Element nach Anspruch 1, **dadurch gekennzeichnet, daß** es aus einem bogenförmigen Abschnitt (3) gebildet ist, dessen Krümmungsradius dergestalt ist, daß der Abschnitt (3) nach seinem Einsetzen in das Auge auf der optischen Achse des Auges zentriert ist.

3. Korrigierendes Element nach Anspruch 1 oder nach Anspruch 2, **dadurch gekennzeichnet, daß** eine hintere Wand (33) die hinteren, voneinander beabstandeten Enden der äußeren Wand (31) und der inneren Wand (32) miteinander verbindet.

4. Element nach einem der Ansprüche 1 bis 3, **dadurch gekennzeichnet, daß** eine Rundung die vorderen, zusammenlaufenden Enden der äußeren Wand (31) und der inneren Wand (32) miteinander verbindet.

5. Korrigierendes Element nach einem der Ansprüche 1 bis 4, **dadurch gekennzeichnet, daß** der von der äußeren Wand (31) und der inneren Wand (32) eingeschlossene Winkel in der Größenordnung von 45° liegt.

6. Korrigierendes Element nach einem der Ansprüche 3 bis 5, **dadurch gekennzeichnet, daß** die hintere Wand (33) abgerundet ist.

7. Korrigierendes Element nach einem der Ansprüche 3 bis 6, **dadurch gekennzeichnet, daß** die hintere Wand (33) durch einen Torusabschnitt gebildet ist.

## Claims

1. An element for the correction of presbyopia, intended for implantation in an eye in a line with the ciliary body, **characterised in that** once it has been put in position in the deep part of the sclera it has, as seen in cross-section, an outer wall (31) parallel with the surface of the sclera (1) and an inner wall (32) parallel with the optical axis (2) of the eye, such that it exerts a centripetal force (F1) on the said ciliary body which is directed perpendicular to the optical axis (2) of the eye.

2. A corrective element according to Claim 1, **characterised in that** it is formed by an arcuate segment (3) whereof the radius of curvature is such that once it has been put in position in the eye the said segment (3) is centred on the optical axis of the said eye.

3. A corrective element according to Claim 1 or Claim 2, **characterised in that** a rear wall (33) connects the ends remote from one another and at the rear of the said outer wall (31) and inner wall (32).

4. A corrective element according to any one of Claims 1 to 3, **characterised in that** a rounded shape connects the concurrent front ends of the said outer wall (31) and inner wall (32).

5. A corrective element according to any one of Claims 1 to 4, **characterised in that** the angle between the outer wall (31) and the inner wall (32) is in the order of 45°.

6. A corrective element according to any one of Claims 3 to 5, **characterised in that** the rear wall (33) is rounded.

7. A corrective element according to any one of Claims 3 to 6, **characterised in that** the rear wall (33) is formed by a torus portion.
